# EUROPEAN PATENT APPLICATION

(11) **EP 3 171 151 A1**
(43) Date of publication of application: **24.05.2017**
(21) Application number: 15822163.0
(22) Date of filing: 25.05.2015
(51) Int. Cl.: G01N 1/10, B04B 5/02, B04B 11/00, G01N 1/28, G01N 33/48, G01N 35/00, G01N 37/00

(54) **DEVICE FOR CONSTANT-VOLUME COLLECTION USING CENTRIFUGATION OR FOR FURTHER STORAGE**

(30) Priority: 18.07.2014 JP 2014147261
(71) Applicant: Shimadzu Corporation, Kyoto-shi, Kyoto 604-8511 (JP)
(72) Inventor: TAKEUCHI Ippei, Kyoto-shi Kyoto 604-8511 (JP); AKECHI Masakazu, Kyoto-shi Kyoto 604-8511 (JP); KANAI Masaki, Kyoto-shi Kyoto 604-8511 (JP); KUDOH Shinobu, Kyoto-shi Kyoto 604-8511 (JP)
(74) Representative: Kilian Kilian & Partner
(86) International application number: PCT/JP2015/064909
(87) International publication number: WO 2016/009720

(57) **Abstract**

A centrifugal separation device including a supporter attached to a centrifuge and a divisional part removable from the supporter is provided. The divisional part has two flow channels and an extracting part. The flow channels extend substantially in parallel to the direction in which centrifugal force acts, and are connected to each other on the leading end side in the direction in which centrifugal force acts. One of the flow channels is a sample flow channel having a sample inlet on the base end side in the direction in which centrifugal force acts, and the other flow channel is an air-discharging flow channel having an outlet on the base end side. The extracting part includes part of the sample flow channel, and has a connecting part for the part to be cut off from the remainder of the sample flow channel. The extracting part allows centrifuged sample components existing in the cut-off part to be extracted.

## Description

### TECHNICAL FIELD

The present invention relates to a device for collecting a liquid or a liquid-state sample containing a plurality of components differing in specific gravity or in which such components are dispersed, such as blood, and thereafter subjecting the sample to a centrifugal operation to collect or store a required fraction by a constant volume.

### BACKGROUND ART

With a conventional centrifuge tube having a capacity of several milliliters or greater, collecting a minute quantity of blood and transferring only supernatant blood plasma components with a micropipette or the like after centrifugal processing while avoiding mixing of blood cell components into the supernatant becomes much more difficult as the quantity of the sample is smaller.

As an apparatus for collecting blood plasma components from a minute quantity of a blood sample, a minute quantity-blood collection tube which is a capillary with its opposite ends opened has been used. In collecting blood plasma components using the minute quantity-blood collection tube, blood is drawn into the minute quantity-blood collection tube. The tip is sealed with putty or the like, and the collection tube is centrifuged. Thereafter, about the interface between the blood plasma part and the blood cell part, the collection tube is broken by being snapped off, and just blood plasma components are extracted. The extracted blood plasma components are analyzed by TLC (thin-layer chromatography), LC (liquid chromatography), LC/MS (liquid chromatography / mass spectrometer), mass spectrometer, or the like.

There is also proposed a centrifuge tube intended to collect only a minute quantity of a white blood cell part which is positioned between a blood cell part and a blood plasma part having been centrifuged (see Patent Document 1). The centrifuge tube includes two upper and lower reservoirs having a great diameter and a large capacity, and a small-capacity reservoir having a small diameter and positioned between the upper and lower reservoirs. The lower large-capacity reservoir is bottomed, and the upper large-capacity reservoir is opened by an opening. Blood is collected from the upper opening by a predetermined quantity, and centrifuged. As a result, the white blood cell part is positioned in the small-capacity reservoir. After the centrifugal processing, a fine glass tube (capillary) is inserted from the upper opening, and the white blood cell components in the small-capacity reservoir are collected.

Studies are also actively conducted in which: several flow channels including capillaries are provided in a disc; the disc is subjected to centrifugal processing whereby blood components are separated; and the blood components are caused to react with a reagent so as to be detected. As a device used for such a scheme, for example, a device formed by a disc-shaped member having integrally formed chamber, flow channels, reservoir, and analysis cells is proposed (see Patent Document 2). A blood sample is introduced into the apparatus and centrifuged so as to separate blood cells from serum. Subsequently, the serum undergoes several processing operations or tests.

### PRIOR ART DOCUMENTS

### PATENT DOCUMENTS

Patent Document 1: Japanese Patent Laid-open Publication No. 01-199159
Patent Document 2 : Japanese Patent Laid-open Publication (Translation of PCT Application) No. 2001-502793

### SUMMARY OF THE INVENTION

### PROBLEMS TO BE SOLVED BY THE INVENTION

In preclinical study conducted in drug development, the quantity of blood obtained from a small animal is limited. Accordingly, it is difficult to collect blood from an individual at every time point required for a pharmacokinetic analysis. Therefore, animals are allocated among blood collection time points after drug administration, so that samples can be collected at different time points from respective animals. Recent sophistication of the apparatuses has drastically improved the measurement sensitivity. Nowadays, just a minute quantity of a sample will suffice for measurement. If blood plasma or serum can be directly and easily obtained by a constant volume required for measurement from a minute quantity of blood, the quantity of blood that must be collected for a measurement can be reduced to a very small quantity. This contributes to a reduction in the number of sacrificed animals. In addition, since a series of samples can be obtained from a single individual, individual difference variations are avoided from measurement data. Further, in clinical trials and clinical settings also, replacing blood collection by a great quantity using a needle, the apparatuses are applicable to easy collection of a sample from a peripheral blood vessel of ears, hands, belly, and the like. This largely contributes to a reduction of burden of blood collection put on infants, children, or patients.

In centrifuging blood using the above-described minute quantity-blood collection tube having its opposite ends opened or the like in place of a centrifuge tube, one end must be sealed with putty or the like. The present invention is directed to samples without being limited to blood, and is directed to every liquid sample containing a plurality of components differing from each other in specific gravity.

An object of the present invention is to provide a centrifugal separation device capable of introducing or drawing in such a sample, centrifuging the sample, and collecting separated predetermined sample components with simple operations.

### SOLUTIONS TO THE PROBLEMS

A centrifugal separation device of the present invention includes: a supporter attached to a centrifuge so that a center of the supporter becomes a center of rotation; and a divisional part disconnectably connected to the supporter or detachably attached to the supporter.

The divisional part has two flow channels and an extracting part. The two flow channels extend substantially in parallel to a direction in which centrifugal force acts, and connected to each other on a leading end side in the direction in which the centrifugal force acts. One of the flow channels is a sample flow channel that has a capacity of storing a sample, and an opening serving as a sample inlet on a base end side in the direction in which the centrifugal force acts. The other flow channel is an air-discharging flow channel formed by a fine tube having an opening serving as an outlet on the base end side in the direction in which the centrifugal force acts.

The extracting part includes part of the sample flow channel, and has a connecting part for cutting off the part of the sample flow channel from a remainder of the sample flow channel. The extracting part allows centrifuged sample components existing in the cut-off part to be extracted.

In order to eliminate work of sealing with putty or the like, in the present invention, the flow channels are connected to each other on the leading end side in the direction in which centrifugal force acts, to form a U-shape. When flow channels are formed into a U-shape being symmetric relative to the direction in which centrifugal force acts, with a blood sample, centrifuged blood plasma components or serum components are branched into the two flow channels of the U-shape. The smaller the flow channel capacity of the air-discharging flow channel relative to the flow channel capacity of the sample flow channel, the smaller the quantity of the sample flowing into the air-discharging flow channel of the flow channels of a U-shape. The quantity of the sample flowing into the air-discharging flow channel is preferably negligible as compared to the quantity of the sample remaining in the sample flow channel. For example, setting the rate of the flow channel capacities to (sample flow channel) : (air-discharging flow channel) = 100 : 1, the sample mostly remains in the sample flow channel. The rate 100 : 1 as the rate between the flow channel capacities is merely an example, and preferably the proportion of the air-discharging flow channel is further smaller.

On the other hand, the proportion of the air-discharging flow channel may be greater than 1/100. In this case, the quantity of the sample existing in the sample flow channel is reduced. When a reduction to some extent is acceptable depending on the quantity of the handled sample, such an air-discharging flow channel can also be employed.

In order to facilitate the work of cutting off the extracting part from the divisional part, the connecting part between the extracting part and the divisional part is preferably formed to be thinner than, narrower than, or thinner and narrower than other part of the divisional part. In this case, the extracting part can be easily cut off from the divisional part by breaking the connecting part with hands or any device.

In one embodiment, the extracting part is disposed at a position on the sample inlet side in the sample flow channel, so as to extract, out of the centrifuged sample components, a sample component whose specific gravity is smaller. For example, in the case where the device of the present invention is used for a blood sample to extract centrifuged blood plasma components or serum components, the sample quantity introduced or drawn into the sample flow channel is adjusted in such a way that centrifuged blood plasma components or serum components will be positioned at the extracting part.

The sample flow channel includes a flow channel connected to the sample inlet, and a downstream flow channel connected to the flow channel. The downstream flow channel may have an inlet whose cross-sectional area is reduced so as to prevent entry of liquid in a state where the centrifugal force does not act. As an example, the sample flow channel may be made up of a first flow channel connected to the sample inlet, a second flow channel connected to the first flow channel, and a third flow channel connected to the second flow channel. In this case, a cross-sectional area of the second flow channel is formed to be smaller relative to a cross-sectional area of the third flow channel so as to prevent a sample in the first flow channel and the second flow channel from entering the third flow channel, in the state where centrifugal force does not act.

In one embodiment, the opening serving as the sample inlet is provided at a surface of the divisional part, and has a size that accepts insertion of a sample introducing device.

In other embodiments, the opening serving as the sample inlet is provided at an end surface of the divisional part. A cross-sectional area of the first flow channel is set to a size that allows a sample to be drawn into the sample inlet by a capillary phenomenon. The first flow channel is provided with an opening at a position opposite to the sample inlet which opening is closed in centrifugation, or a cross-sectional area of the air-discharging flow channel is set to be equal to or greater than the cross-sectional area of the first flow channel. In this case, in order for a sample to be smoothly drawn in by the capillary phenomenon, preferably an inner surface and the opening serving as the sample inlet of the first flow channel are rendered hydrophilic.

The divisional part is a joined body made up of a base member and a cover member. The sample flow channel and the air-discharging flow channel may be formed at a joining interface of the base member and the cover member.

Further, in the case where the divisional part is a joined body made up of a base member and a cover member, and the sample flow channel and the air-discharging flow channel are formed at a joining interface of the base member and the cover member, a space may be formed adjacent to part of a surrounding of the third flow channel so that joining strength of the joining interface at the part of the surrounding of the third flow channel becomes weaker than joining strength of the joining interface at other part of the surrounding of the third flow channel. Providing such a portion with weak joining strength allows the portion with weak joining strength to break when freeze-stored, whereby the third flow channel is prevented from greatly damaged.

In one embodiment, the supporter is disc-like, a plurality of the divisional parts being radially disposed and connected onto a circumference of the disc. A portion connecting between the supporter and each of the divisional parts is formed to be thinner than, narrower than, or thinner and narrower than other part of each of the divisional parts.

In other embodiments, the supporter is disc-like, on a surface of which disc recesses are formed for a plurality of the divisional parts to be respectively radially disposed and attached by being fitted into. The divisional parts and the recesses may be shaped so as to prevent the divisional parts from shifting in a centrifugal force direction when the centrifugal force acts.

In an embodiment in which recesses are formed at the supporter for the divisional parts to be attached by being fitted into, the supporter may be provided with a hole at a position corresponding to the extracting part when each of the divisional parts is attached. By disposing a container below the hole and disconnecting the extracting part from the divisional part from above the extracting part with hands or any device, the extracting part having been cut off from the divisional part can be received by the container and carried to an analysis apparatus.

With a minute quantity-blood collection tube, a container or the like must be separately provided for the purpose of distinguishing from other samples or for protecting the minute quantity-blood collection tube from damage when freeze-stored. When a multitude of samples are handled, the space for storing the separately provided containers may not be negligible. In particular, when the storage space is limited, such as when a freezer is used, it is a critical problem. In a preferred embodiment of the present invention solving such a problem, the supporter and the divisional parts are disc-like in a state where the divisional parts are mounted or attached to the supporter, and shaped such that a plurality of the discs can be stored as being stocked. This reduces the space for storage.

### EFFECTS OF THE INVENTION

According to the present invention, without the necessity of sealing with putty or the like, a minute quantity of a sample can be centrifuged, whereby predetermined separated components can be extracted.

### BRIEF DESCRIPTION OF THE DRAWINGS

Fig. 1 is a diagram showing a first embodiment in the state where divisional parts are mounted to a supporter, in which Fig. 1(A) is a plan view and Fig. 1(B) is a front view.
Fig. 2 is a diagram showing the detached divisional part and extracting part according to the first embodiment, in which Fig. 2 (A) is a plan view of the divisional part, Fig. 2(B) is a front view of the divisional part, Fig. 2(C) is a plan view of one disconnected extracting part, and Fig. 2(B) is a front view of one disconnected extracting part.
Fig. 3 is a diagram showing a second embodiment in the state where divisional parts are attached to a supporter, in which Fig. 3(A) is a plan view and Fig. 3(B) is a front view.
Fig. 4 is a diagram showing the divisional part according to the second embodiment, in which Fig. 4 (A) is a plan view and Fig. 4(B) is a front view.
Fig. 5 is a diagram showing the supporter according to the second embodiment, in which Fig. 5(A) is a plan view and Fig. 5(B) is a front view.
Fig. 6 is a plan view of the divisional part and an extracting part according to the second embodiment, showing operations from drawing a sample into the divisional part to cutting off the extracting part.
Fig. 7 is a diagram showing a divisional part according to a third embodiment, in which Fig. 7(A) is a plan view and Fig. 7(B) is a front view.

### EMBODIMENTS OF THE INVENTION

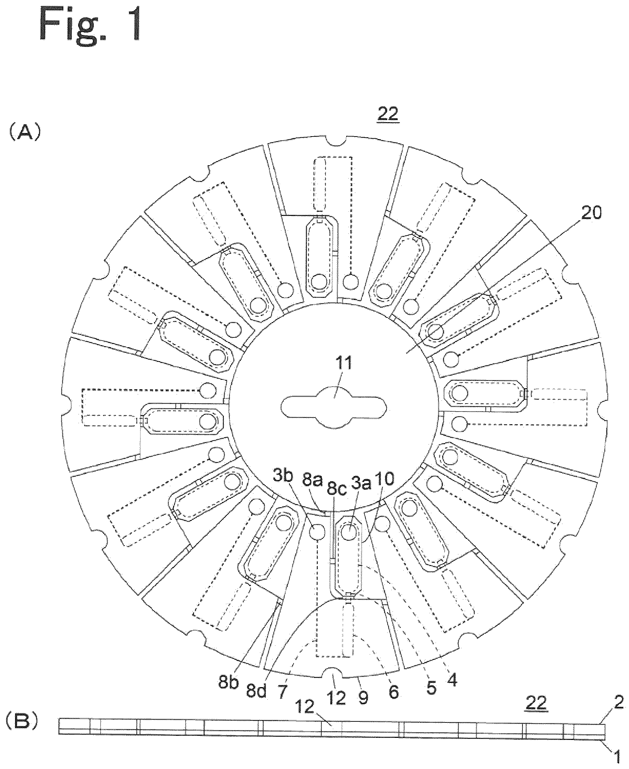

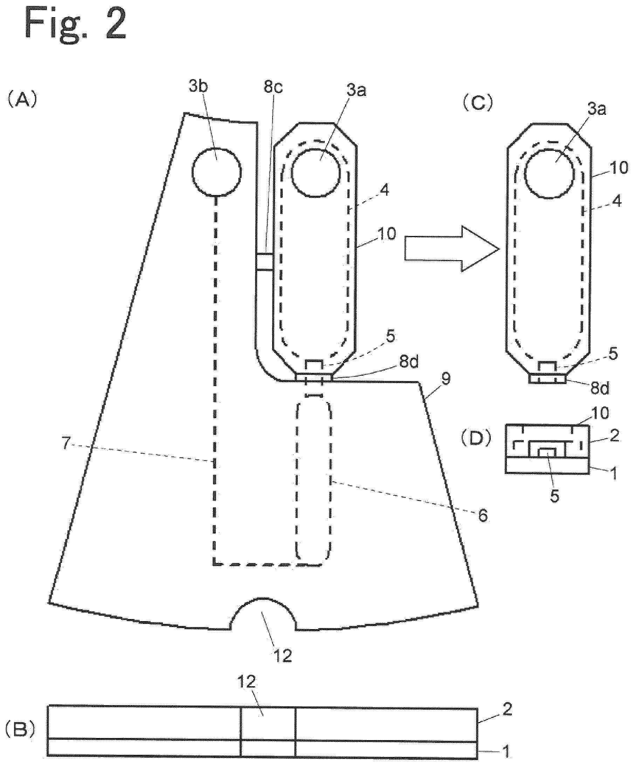

Figs. 1 and 2 show a minute quantity-centrifugal separation device according to a first embodiment.

A minute quantity-centrifugal separation device (hereinafter simply referred to as a separation apparatus in embodiments) 22 is disc-like as a whole and structured by a base 1 and a cover 2. At the center, a disc-like supporter 20 is formed. On the circumference of the disc of the supporter 20, a plurality of divisional parts 9 are disposed radially from the supporter 20. While the number of divisional parts 9 is not particularly limited, the divisional parts 9 are provided by 12 pieces in the present embodiment.

The separation apparatus 22 is structured by, for example, a resin material. While the resin material is not particularly limited, it may be COP (cyclo-olefin polymers), PMMA (polymethyl methacrylate resin), PP (polypropylene resin), PC (polycarbonate resin) or the like.

The base 1 and the cover 2 are integrated with each other by being joined to each other, to structure the separation apparatus 22. While the method of the joining also is not particularly limited, for example, the base 1 and the cover 2 may be bonded to each other by heating, or respective joining surfaces of the base 1 and the cover 2 may be irradiated with plasma and thereby activated, and bonded to each other.

The divisional parts 9 are each coupled to supporter 20 by a connecting part 8a. Adjacent divisional parts 9 are also connected by a connecting part 8b. The divisional parts 9 are each provided with an extracting part 10. The divisional part 9 and corresponding extracting part 10 are coupled to each other by connecting parts 8c, 8d provided at two positions. The connecting parts 8a to 8d are formed to be thinner and narrower than other portions so that they can be disconnected by being snapped off.

Between the supporter 20 and the divisional parts 9, the divisional parts 9 can be cut off from the supporter 20 by disconnecting the connecting parts 8a. The divisional parts 9 can be cut off from one another by disconnecting each connecting part 8b between adjacent divisional parts 9. Figs. 2 (A) and 2(B) show one divisional part 9 cut off from both the supporter 20 and the adjacent divisional parts 9.

By disconnecting the connecting parts 8c and 8d at two positions between the divisional part 9 and the extracting part 10, the extracting part 10 can be cut off from the divisional parts 9. Figs. 2(C) and 2(D) show the extracting part 10 cut off from the divisional part 9.

The supporter 20, the divisional part 9, and the extracting part 10 are each a joined body made up of the base 1 and the cover 2. Out of the connecting parts 8a to 8d, the connecting part 8d coupling between the divisional part 9 and the extracting part 10 has a second flow channel 5 formed therein, which will be described later, and is also a joined body made up of the base 1 and the cover 2. On the other hand, since other connecting parts 8a to 8c just serve to couple between the members while being capable of being snapped off, the connecting parts 8a to 8c may each be a joined body made up of the base 1 and the cover 2 or may be made of just the base 1 or the cover 2.

At the center of the supporter 20, a through hole 11 formed by a circular hole and an oval hole connecting thereto and extending in one direction is formed. Along the outer periphery of the divisional parts 9, cutouts 12 are formed. The hole 11 and the cutouts 12 are for attaching the separation apparatus 22 to the rotor of a centrifuge. The shape of the hole 11 or the cutouts 12 is not particularly limited to the shape shown in Fig. 1(A) so long as it enables attachment of the separation apparatus 22 to the centrifuge. When the separation apparatus 22 is attached to the centrifuge and subjected to centrifugal processing, the radial direction extending outward from the center of the hole 11 is the direction in which the centrifugal force acts.

As shown in Fig. 2 (A), the planar shape of the divisional part 9 with the extracting part 10 coupled thereto is a shape of a sector from which the central portion is removed, and is similar to a trapezoid. The sector has its narrow width portion coupled to the supporter 20 by the connecting part 8a. In connection with the divisional part 9 and the extracting part 10, the base 1 is a flat plate; a recess structuring a flow channel is formed on the inner side of the cover 2; and the cover 2 is further provided with holes that respectively serve as an inlet 3a for introducing a sample and as an outlet 3b for discharging air inside the flow channel in centrifugal processing.

The inlet 3a is formed at the extracting part 10. The outlet 3b is formed at the divisional part 9. The inlet 3a and the outlet 3b are substantially equidistant from the center of the separation apparatus 22. The inlet 3a has a size that allows a sample to be introduced using any device such as a pipet. In the divisional part 9 and the extracting part 10, continuous flow channels 4, 5, and 6 are formed to extend outward from the inlet 3a in the substantially radial direction of the separation apparatus 22, and a flow channel 7 is formed to extend outward from the outlet 3b in the substantially radial direction of the separation apparatus 22. The flow channels extending in the radial direction are coupled to each other by part of the flow channel 7 on the circumferential side of the separation apparatus 22, to form a series of flow channels. The series of flow channels is substantially U-shaped, having the inlet 3a and the outlet 3b on the central side of the separation apparatus 22 and connected on the circumferential side.

The extracting part 10 has a shape extending in the radial direction of the separation apparatus 22. In the extracting part 10, the first flow channel 4 is formed in a shape extending in the radial direction of the separation apparatus 22, and the inlet 3a is disposed on the base end side of the first flow channel 4, that is, disposed on the central side of the separation apparatus 22. In the divisional part 9, the third flow channel 6 is formed. The third flow channel 6 is disposed on the extension line in the extending direction of the first flow channel 4 , and is formed into a shape extending in the radial direction of the separation apparatus 22. The first flow channel 4 and the third flow channel 6 are coupled to each other by the second flow channel 5 passing in the connecting part 8d.

In the divisional part 9, the fourth flow channel 7 is formed. The fourth flow channel 7 is connected to the third flow channel 6 on the leading end side of the third flow channel 6, that is, on the outer side in the radial direction of the separation apparatus 22. The fourth flow channel 7 is formed to be bent on the peripheral side of the separation apparatus 22 and connected to the outlet 3b.

The first flow channel 4 has the greatest cross-sectional area, and the third flow channel 6 has the second greatest cross-sectional area. The cross-sectional area of the second flow channel 5 is smaller than the cross-sectional area of the third flow channel 6. The cross-sectional area of the fourth flow channel 7 is the smallest.

The first flow channel 4 is a flow channel for introducing a liquid sample from the inlet 3a. The cross-sectional area of the third flow channel 6 is set to be greater relative to the cross-sectional area of the second flow channel 5 so that the liquid sample introduced into the first flow channel 4 stays in the first flow channel 4 and the second flow channel 5 and does not flow into the third flow channel 6 in the state before the separation apparatus 22 is set on the centrifuge and subjected to centrifugal processing. In order for the liquid to flow into the third flow channel 6 from the second flow channel 5, force for the liquid to spread from the second flow channel 5 into the third flow channel 6 is necessary. Therefore, as the difference in the cross-sectional area between the second flow channel 5 and the third flow channel 6 is greater, the liquid can be stably stopped from flowing into the third flow channel 6 from the second flow channel 5.

Here, exemplary dimensions of the separation apparatus 22 according to the embodiment are shown. However, it goes without saying that the present invention is not limited to the embodiment of such dimensions.

The diameter of the outer shape of the whole separation apparatus 22 is 104 mm. The base 1 has a thickness of 1 mm, and the cover 2 has a thickness of 2 mm. The first flow channel 4 has a width of 4 mm, a depth of 1 mm, a length of 13.5 mm, and its planar shape is oval or rectangular. The second flow channel 5 has a width of 1 mm, a depth of 0.5 mm, a length of 2 mm, and its planar shape is rectangular. The third flow channel 6 has a width of 2 mm, a depth of 1 mm, a length of 10.5 mm, and its planar shape is oval or rectangular. The fourth flow channel 7 has a width of 0.01 mm, a depth of 0.01 mm, and a length of 30 mm, and is capillary. The inner surface of the flow channels 4, 5, 6, and 7 has been rendered hydrophilic by plasma irradiation.

Next, a description will be given of the processing operation of a blood sample using the separation apparatus 22 according to the present embodiment. Here, the description will be given of the case where blood plasma or serum is collected from a blood sample by centrifugal separation. In the case where blood plasma is collected, anticoagulant such as heparin is added to blood. In the case where serum is collected, no anticoagulant is added. Here, while an operation of collecting blood plasma will be described, the operation is the same also in the case where serum is collected.

Blood is collected as a sample by 40µL with a micropipette or the like, and introduced into the first flow channel 4 from the inlet 3 formed at the extracting part 10. The introduced blood flows through the first flow channel 4 to reach the second flow channel 5 and stops. Since the separation apparatus 22 is provided with a plurality of extracting parts 10, blood samples as many as the number of the extracting parts 10 can be processed. All the extracting parts 10 are not necessarily used. Blood samples should be extracted to the extracting parts 10 just in the required number corresponding to the number of the blood samples to be processed.

After blood is introduced to the extracting parts 10 in the required number, the separation apparatus 22 is attached to the rotor of the centrifuge, and centrifugal separation is performed by causing the rotor to rotate. In Fig. 1(A), the rotor rotates clockwise. While the conditions of the centrifugal separation are, for example, a rotation speed of 10,000 rpm and a time of 5 minutes, other conditions may be employed so long as blood cells and blood plasma separate from each other.

After the centrifugal separation, approximately half the volume (hematocrit) of the blood is obtained as blood cell components. In the present embodiment, since the capacity of the third flow channel 6 is designed to be approximately 20 µL, the blood cell components gather in the third flow channel 6, and the blood plasma components gather in the first flow channel 4.

In the centrifugal separation, the blood flows into the third flow channel 6, and further flows into the fourth flow channel 7. By the blood flowing into the third flow channel 6 and the fourth flow channel 7, the air having been in these flow channels is discharged from the outlet 3b through the fourth flow channel 7. Since the fourth flow channel 7 is intended to discharge the air in the flow channels so that the centrifugal separation is smoothly performed, preferably the capacity of the fourth flow channel 7 is as small as possible. In the present embodiment, since the fourth flow channel 7 is formed to be capillary, the capacity of the fourth flow channel 7 is very small as compared to the total capacity of the first to third flow channels 4 to 6, and set to be 1/100 as great as the total capacity of the first to third flow channels 4 to 6 or smaller. Accordingly, the quantity of the blood flowing into the fourth flow channel 7 is negligible as compared to the quantity of blood remaining in the flow channels 4 to 6.

Further, since the inlet 3a and the outlet 3b are substantially equidistant from the center of the separation apparatus 22, and the blood in the first flow channel 4 shifts to the position distanced from the inlet 3a in the centrifugal direction, the blood flowing into the fourth flow channel 7 will not reach the outlet 3b. Accordingly, the blood flowing into the fourth flow channel 7 will not be discharged from the outlet 3b.

After the centrifugal separation, in the case where the separated blood plasma components are provided as a sample for analysis or the like, the connecting parts 8c and 8d are snapped off and disconnected, so that the extracting part 10 is detached from the divisional part 9. In order to extract the blood plasma components from the first flow channel 4 exposed at the end surface of the extracting part 10 on the connecting part 8d side, an absorbing member such as a filter paper should be pressed against the exposed portion. The blood plasma components extracted into the absorbing member can be used as a sample for analysis. Further, the blood plasma components can also be drawn out from the inlet 3 formed at the extracting part 10.

When the connecting part 8d is disconnected, the blood in the second flow channel 5 having been formed in the connecting part 8d is discarded. However, in the present embodiment, the fourth flow channel 7 is designed to have a capacity of less than 1 µL, so as to reduce the quantity of discarded blood.

In the case where just part of extracting parts 10 of the separation apparatus 22 is used, only the divisional parts 9 having the used extracting parts 10 are detached from the separation apparatus 22 and the divisional parts 9 having unused extracting parts 10 are left at the separation apparatus 22. Thus, the separation apparatus 22 with the unused extracting parts 10 can be used for centrifugal processing of other samples.

There is also the case where the blood sample after centrifugal separation is not subjected to analysis but freeze-stored. In this case, the separation apparatus 22 in the state coupled to the divisional parts 9 and the extracting parts 10 can be freeze-stored as it is. The separation apparatus 22 does not require a separate container or the like for protecting the extracting parts 10. Further, since the separation apparatus 22 is formed disc-like, a plurality of separation apparatuses 22 can be stored as being stacked in freeze-storing. Thus, the separation apparatus 22 is advantageous in requiring just a small space for storage.

Further, since the separation apparatus 22 is provided with a plurality of extracting parts 10, a plurality of samples per separation apparatus 22 can be freeze-stored. Stacking and storing a plurality of separation apparatus 22 is also advantageous in that many samples after centrifugal separation can be stored in a small space.

Still further, it is also possible that, after centrifugal separation, the divisional part 9 coupled to the extracting part 10 is detached from the separation apparatus 22 as shown in Fig. 2 (A), and the divisional part 9 coupled to the extracting part 10 can be freeze-stored. The divisional part 9 coupled to the extracting part 10 also does not require a separate container or the like for protecting the extracting part 10. Further, since the divisional part 9 coupled to the extracting part 10 is flat, it can be stored as being stacked, and advantageous in requiring just a small space for storage.

The divisional part 9 has a space for placing an identification label, such as a barcode label, for identifying the sample. Accordingly, in the case where the divisional part 9 is stored in the state being coupled to the extracting part 10 also, a separate container or the like for placing an identification label is not necessary.

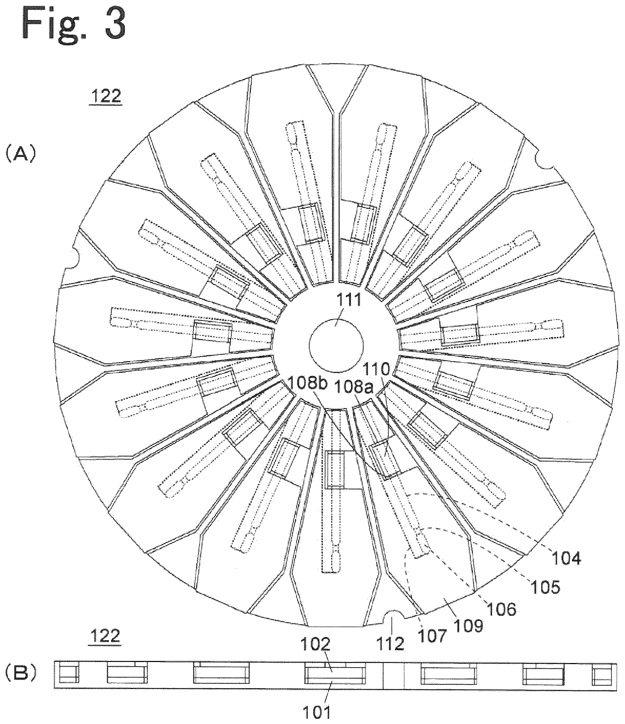

Figs. 3 to 6 show a separation apparatus according to a second embodiment.

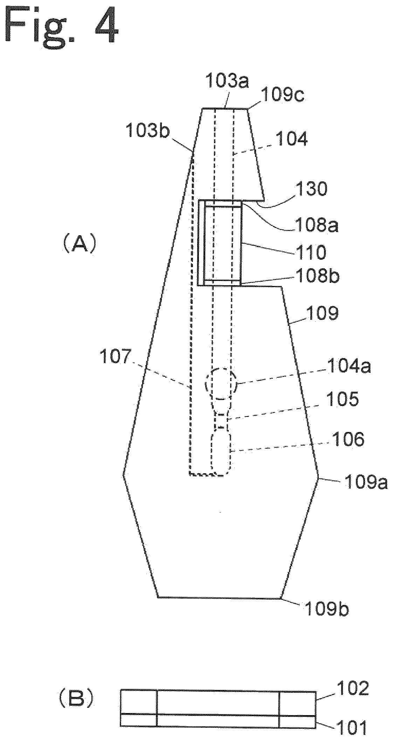

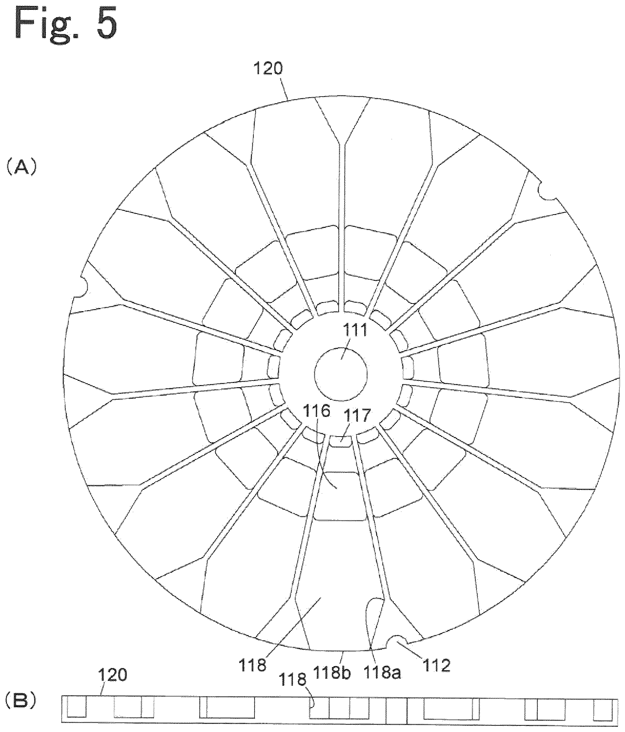

A separation apparatus 122 according to the present embodiment is made up of a supporter 120 shown in Fig. 5, and divisional parts 109 shown in Fig. 4. The supporter 120 holds the divisional parts 109, and is attached to a centrifuge and subjected to centrifugal processing.

Each divisional part 109 is made up of a base 101 and a cover 102. The planar shape of the divisional part 109 is formed by an intermediate part 109a, a leading end part 109b, and a base end part 109c, in which the width of the intermediate part 109a is the greatest; the width of the leading end part 109b is smaller than the width of the intermediate part 109a; and the width of the base end part 109c is further smaller than the width of the leading end part 109b. Here, when the divisional part 109 is placed on the supporter 120, the side oriented to the center of the supporter 120 is referred to as the base end side, and the side opposite thereto is referred to as the leading end side.

The shape from the intermediate part 109a to the leading end part 109b is a trapezoid whose long side is the width of the intermediate part 109a. The shape from the intermediate part 109a to the base end part 109c is, as a whole, a trapezoid whose long side is the width of the intermediate part 109a, and in part of the trapezoid, a cutout 130 for providing an extracting part 110 is provided.

The divisional part 109 is structured by the base 101 and the cover 102 being integrated with each other by being joined to each other. The divisional part 109 is structured by a resin material, for example. While the resin material is not particularly limited, it may be COP, PMMA, PP, PC or the like similarly to the first embodiment.

While the method of joining the base 101 and the cover 102 also is not particularly limited, similarly to the first embodiment, for example, the base 101 and the cover 102 may be bonded to each other by heating, or respective joining surfaces of the base 101 and the cover 102 may be irradiated with plasma and thereby activated, and bonded to each other.

In the divisional part 109, a first flow channel 104, a second flow channel 105, and a third flow channel 106 which are continuous and extending from the base end part 109c to the leading end part 109b are formed. The first flow channel 104 is formed over the length that passes through the extracting part 110 formed at the cutout 130 to reach the intermediate part 109a of the divisional part 109. Further, the first flow channel 104 has an opening at the end surface of the base end part 109c, which opening serves as an inlet 103a for drawing in a sample.

The first flow channel 104 draws in a liquid sample from the inlet 103a by means of the capillary phenomenon. Accordingly, the cross-sectional area of the first flow channel 104 is set to be small enough to cause the capillary phenomenon to occur. Further, since the resin material exemplarily noted above is hydrophobic, preferably at least the inner surface of the first flow channel 104 and the inlet 103a that draw in a sample by means of the capillary phenomenon are treated to become hydrophilic. Such a hydrophilic treatment may be plasma irradiation, or coating with heparin, EDTA (ethylenediaminetetraacetic acid) or the like.

In the divisional part 109, a fourth flow channel 107 that extends substantially in parallel to the flow channels 104 to 106 is further formed. The fourth flow channel 107 is connected to the third flow channel 106 at the leading end side of the third flow channel 106. The fourth flow channel 107 is bent on the leading end side, extends to the side surface on the base end side and opens at the side surface. This opening serves as an outlet 103b for discharging air. The outlet 103b is disposed on the base end part side relative to blood in the first flow channel 104 after centrifugal separation, so that the blood will not exit from the outlet 103b via the fourth flow channel 107 in the centrifugal processing.

As compared to the cross-sectional area of the first flow channel 104 and that of the third flow channel 106, the cross-sectional area of the second flow channel 105 is smaller, and the cross-sectional area of the fourth flow channel 107 is the smallest. Similarly to the first embodiment, the fourth flow channel 107 is formed to be capillary, and the capacity of the fourth flow channel 107 is very small as compared to the total capacity of the first to third flow channels 104 to 106, and set to be 1/100 as great as the total capacity of the first to third flow channels 104 to 106 or smaller.

The first flow channel 104 draws in a liquid sample from the inlet 103a by means of the capillary phenomenon, and is designed to have a size with which capillary force capable of drawing in liquid occurs. The cross-sectional area of the third flow channel 106 is set to be greater relative to the cross-sectional area of the second flow channel 105, so that a liquid sample drawn in the first flow channel 104 stays in the first flow channel 104 and the second flow channel 105 and will not flow into the third flow channel 106, in the state before the separation apparatus 122 is attached to a centrifuge and subjected to centrifugal processing. This is similar to the first embodiment.

The first flow channel 104 extends in the extracting part 110 provided at the cutout 130 of the divisional part 109. Since the extracting part 110 is for collecting blood plasma or serum, the extracting part 110 is at a position where blood plasma or serum exists in the first flow channel 104 after centrifugal processing.

The extracting part 110 is coupled to the divisional part 109 by a connecting part 108a on the base end side and coupled to the divisional part 109 by a connecting part 108b on the leading end side, in the cutout 130.

The connecting parts 108a and 108b are preferably formed to be thinner than other parts so that they can be easily disconnected. By the connecting parts 108a and 108b being disconnected, the extracting part 110 can be detached from the divisional part 109.

As shown in Figs. 5(A) and 5(B), the supporter 120 is a disc-like plate provided with a plurality of recesses 118 for the divisional parts 109 to be fitted and held thereby. The number of the recesses 118 is not particularly limited. While the number of the recesses 118 is fifteen in the present embodiment, it can be arbitrarily determined. The supporter 120 being capable of holding a greater number of the divisional parts 109 is advantageous because a greater number of divisional parts 109 can be processed in a single centrifugal separation operation. It is also advantageous when the supporter 120 holding the divisional parts 109 is stored because a greater number of the divisional parts 109 can be stored in a smaller space.

In order for the supporter 120 to be attached to a centrifuge, a hole 111 is formed at the center of the supporter 120 and a plurality of cutouts 112 are formed at the periphery of the supporter 120. While the shape of the hole 111 is circular herein, it may have the shape as in the first embodiment. Further, the number of the cutouts 112 is not particularly limited.

The planar shape of each recess 118 of the supporter 120 corresponds to the shape of each divisional part 109. The width of an intermediate part 118a is the greatest, and the width becomes smaller toward a leading end part 118b. The intermediate part 118a corresponds to an intermediate part 109a of the divisional part 109, and the leading end part 118b corresponds to a leading end part 109b of the divisional part 109. Figs. 3(A) and 3(B) show the state where the divisional parts 109 are fitted into the recesses 118 of the supporter 120 and held thereby.

In the state where the divisional parts 109 are held by the supporter 120, when the supporter 120 is attached to the centrifuge and rotated, the centrifugal force acts on the divisional parts 109 outward from the center of rotation. Here, by virtue of the small width of the leading end part 118a of each recess 118 of the supporter 120, the divisional parts 109 will not be thrown out from the supporter 120 in the centrifugal force direction. In this manner, the divisional parts 109 and the recesses 118 are shaped to prevent the divisional parts 109 from shifting in the centrifugal force direction when the centrifugal force acts thereon.

The depth of the recesses 118 may be smaller than the thickness of the divisional parts 109 so long as the divisional parts 109 have a thickness with which the divisional parts 109 will not come off from the recesses 118 of the supporter 120. In the present embodiment, in order to reduce the risk of the divisional parts 109 coming off from the recesses 118 of the supporter 120 during centrifugal processing, the depth of the recesses 118 is greater than the thickness of the divisional parts 109. As the depth of the recesses 118 is greater than the thickness of the divisional parts 109, when the supporters 120 each holding the divisional parts 109 are stacked in the thickness direction, a greater wasteful space is formed between the divisional parts 109 and the bottom surface of the supporter 120 stacked immediately above. Accordingly, preferably the depth of the recesses 118 is equal to the thickness of the divisional parts 109 or slightly greater.

In the present embodiment, a through hole 116 is formed at a site of the supporter 120 corresponding to the extracting part 110 when the divisional part 109 is held in the recess 118. The through hole 116 preferably has a size that includes the extracting part 110 and two connecting parts 108a and 108b provided at its opposite ends. The through hole 116 is used in detaching the extracting part 110 from the divisional part 109 while the divisional part 109 is held by the supporter 120 after centrifugal processing. In the state where the supporter 120 is holding the divisional part 109, the user presses a tool, which is fabricated to disconnect the two connecting parts 108a and 108b by pressing from above, against the supporter 120 holding the divisional part 109. Alternatively, the user attaches the supporter 120 to an apparatus having such a tool. In such an operation, using the hole 116, the connecting parts 108a and 108b are disconnected and the extracting part 110 is detached from the divisional part 9. Such a tool or an apparatus is preferably fabricated to be capable of detaching a plurality of extracting parts 110 at once.

Further, at the base end part of the recess 118, a recess 117 is formed at a site corresponding to the base end part 109c of the divisional part 109. This is for preventing the supporter 120 from being smeared with blood, which may be remaining near the base end part 109c because the blood is drawn in from the inlet 103a of the first flow channel 104 of the divisional part 109 at the base end part 109c.

While the material of the supporter 120 is not particularly limited, it may be identical to that of the divisional parts 109.

The exemplary dimensions of the separation apparatus 122 according to the embodiment will be shown. However, it goes without saying that the present invention is not limited to the embodiment of such dimensions. The base 101 of the divisional parts 109 has a thickness of 1 mm, and the cover 102 thereof has a thickness of 2 mm. The first flow channel 104 has, for example, a width of 1.6 mm and a depth of 1 mm, so as to be capable of drawing in liquid by capillary force. While the length should be determined based on the blood quantity to be drawn in, an exemplary length is 25 mm. The second flow channel 105 has a width of 1 mm, a depth of 0.5 mm, and a length of 1 mm. The third flow channel 106 has a width of 1.6 mm, a depth of 1 mm, and a length of 4 mm. The fourth flow channel 107 has a width of 0.01 mm, a depth of 0.01 mm, and a length of 30 mm. The surface of each flow channel has been rendered hydrophilic by plasma irradiation.

In the divisional part 109, when the cross-sectional area of the first flow channel 104 is set to be smaller than that of the third flow channel 106, the second flow channel 105 may be dispensed with. In other words, the third flow channel 106 may be directly connected to the first flow channel 104. At the connecting part between the first flow channel 104 and the third flow channel 106, the cross-sectional area of the flow channel increases from the first flow channel 104 toward the third flow channel 106. Accordingly, depending on the degree of the increase, blood drawn in by the capillary phenomenon stops at the connecting part, and will not flow into the third flow channel 106.

As indicated by a broken line in Fig. 4, in the divisional part 109, an opening 104a may be provided at the first flow channel 104 so as to facilitate blood collection by the capillary phenomenon. The opening 104a is preferably positioned opposite to the inlet 103a and near the second flow channel 105. In the case where the opening 104a is provided, it must be closed during centrifugal separation. For example, a sticker may be placed over the opening 104a so as to close the opening 104a in centrifugal separation. Further, for example, when the divisional part 109 is attached to the supporter 120, the side provided with the opening 104a may face the supporter 120. The supporter 120 may be provided with a projection at a position corresponding to the opening 104a of the attached divisional part 109, so that the projection closes the opening 104a.

In the divisional part 109, in order to facilitate blood collection by the capillary phenomenon, the cross-sectional area of the fourth flow channel 107 being the air-discharging flow channel may be increased. When importance is placed on easier blood collection by the capillary phenomenon, the cross-sectional area of the fourth flow channel 107 should be increased to be equal to or greater than that of the first flow channel 104. On the other hand, an increase in the cross-sectional area of the fourth flow channel 107 increases wasteful blood quantity because blood enters also the fourth flow channel 107 in centrifugal separation. In the case where the blood sample is in a great quantity, an embodiment with an increased cross-sectional area of the fourth flow channel 107 is effective.

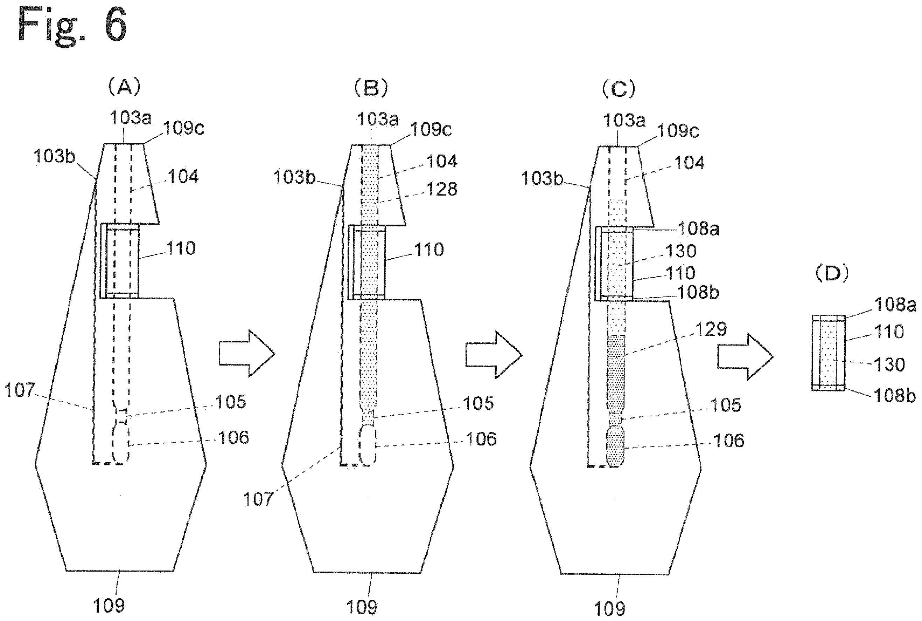

Next, with reference to Fig. 6, a description will be given of a blood sample processing operation using the separation apparatus 122 according to the present embodiment. Again, while the description will be given of the case where blood plasma is collected from a blood sample by centrifugal separation, the description holds true for the case where serum is collected.
(A) In drawing in blood, the divisional part 109 is taken out from the supporter 120.
(B) As a sample, blood 128 is drawn in by 40 µL. The quantity of drawn-in blood is determined by the capacity of the first flow channel 104 and the second flow channel 105. When the inlet 103a exposed at the end surface on the base end side of the divisional part 109 is brought into contact with blood, the blood is drawn into the first flow channel 104 by the capillary phenomenon. When the second flow channel 105 is filled with the drawn-in blood 128, the drawing stops.
(C) The divisional part 109 is returned to the supporter 120, and undergoes centrifugal separation with a centrifuge. By the centrifugal separation, the blood is separated into blood cell components 129 and blood plasma components 130.
(D) With hands or using any tool or apparatus, the extracting part 110 is disconnected at the disconnecting parts 108a and 108b at its opposite ends, and detached from the divisional part 109. The extracting part 110 is put into a predetermined container of an analysis apparatus. The container is attached to the analysis apparatus and analysis is performed.

In the present embodiment also, the divisional part 109 after the centrifugal processing can be freeze-stored as being held by the supporter 120. Since the supporter 120 holding the divisional parts 109 is also disc-like, the supporter 120 holding the divisional parts 109 can be stored as being stacked.

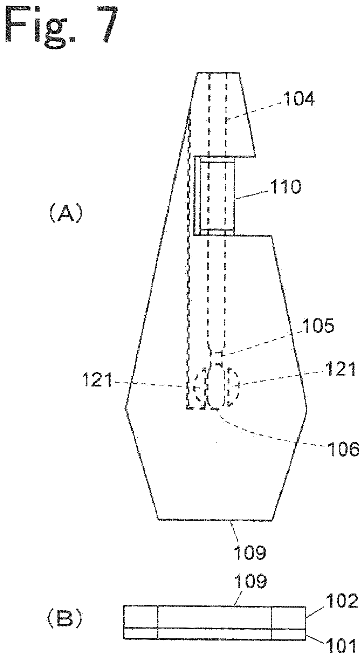

Fig. 7 shows a third embodiment. When the divisional part 9 according to the first embodiment is freeze-stored, in some cases, the joining interface between the base 1 and the cover 2 may unjoin near the third flow channel 6. This is considered to be the phenomenon caused by liquid in the second flow channel 5 and the fourth flow channel 7 being smaller in the cross-sectional area than the third flow channel 6 freezing earlier than liquid in the third flow channel 6, resulting in an increase in the volume of liquid in the third flow channel 6. This phenomenon is also observed with the divisional part 109 according to the second embodiment.

Accordingly, in order to control such unjoining of the joining interface, as shown in Fig. 7, spaces 121 are formed near the third flow channel 106 within a distance of, for example, 1 mm. In the present embodiment, while the spaces 121 are formed on both sides of the third flow channel 106, the space 121 may be disposed only on one side of the third flow channel 106. Forming the spaces 121 near the third flow channel 106 weakens the strength of the joining interface between the third flow channel 106 and the spaces 121 as compared to other parts around the third flow channel 106. Therefore, when the liquid in the third flow channel 106 freezes, the joining interface between the third flow channel 106 and the spaces 121 selectively unjoins. Thus, unjoining at the joining interface can be prevented from occurring at other portions around the third flow channel 106.

As the joining interface between the third flow channel 106 and the spaces 121 unjoins, liquid in the third flow channel 106 flows into the spaces 121, and the liquid is wasted. Accordingly, the spaces 121 are designed to have a minute capacity, such as a depth of 0.01 mm, a width of 1 mm, and a length of 3.5 5 mm. Thus, even if blood cells of a sample or the like flow into the spaces 121, the waste can be reduced to a minimum.

### DESCRIPTION OF REFERENCE SIGNS

1, 101: Base
2, 102: Cover
3a, 103a: Inlet
3b, 103b: Outlet
4, 104: First flow channel
5, 105: Second flow channel
6, 106: Third flow channel
7, 107: Fourth flow channel (Air-discharging flow channel)
8a to 8d, 108a, 108b: Connecting part
9, 109: Divisional part
10, 110: Extracting part
20, 120: Supporter
22, 122: Separation apparatus
116: Hole
118: Recess

## Claims

1. A centrifugal separation device comprising:
a supporter attached to a centrifuge so that a center of the supporter becomes a center of rotation; and
a divisional part disconnectably connected to the supporter or detachably attached to the supporter, wherein
the divisional part has two flow channels and an extracting part,
the two flow channels extend substantially in parallel to a direction in which centrifugal force acts, and connected to each other on a leading end side in the direction in which the centrifugal force acts,
one of the flow channels is a sample flow channel that has a capacity of storing a sample, and an opening serving as a sample inlet on a base end side in the direction in which the centrifugal force acts,
the other flow channel is an air-discharging flow channel that has an opening serving as an outlet on the base end side in the direction in which the centrifugal force acts, and
the extracting part includes part of the sample flow channel, and has a connecting part for cutting off the part of the sample flow channel from a remainder of the sample flow channel, the extracting part allowing centrifuged sample components existing in the cut-off part to be extracted.

2. The centrifugal separation device according to claim 1, wherein the connecting part is formed to be thinner than, narrower than, or thinner and narrower than other part of the divisional part.

3. The centrifugal separation device according to claim 1 or 2, wherein the extracting part is disposed at a position on the sample inlet side in the sample flow channel, so as to extract, out of the centrifuged sample components, a sample component whose specific gravity is smaller.

4. The centrifugal separation device according to any one of claims 1 to 3, wherein
the sample flow channel includes a flow channel connected to the sample inlet, and a downstream flow channel connected to the flow channel, and
the downstream flow channel has an inlet whose cross-sectional area is reduced so as to prevent entry of liquid in a state where the centrifugal force does not act.

5. The centrifugal separation device according to claim 4, wherein
the sample flow channel is made up of a first flow channel connected to the sample inlet, a second flow channel connected to the first flow channel, and a third flow channel connected to the second flow channel, and
a cross-sectional area of the second flow channel is formed to be smaller relative to a cross-sectional area of the third flow channel so as to prevent a sample in the first flow channel and the second flow channel from entering the third flow channel, in the state where the centrifugal force does not act.

6. The centrifugal separation device according to any one of claims 1 to 5, wherein the opening serving as the sample inlet is provided at a surface of the divisional part, and has a size that accepts insertion of a sample introducing device.

7. The centrifugal separation device according to any one of claims 1 to 5, wherein
the opening serving as the sample inlet is provided at an end surface of the divisional part,
a cross-sectional area of the first flow channel is set to a size that allows a sample to be drawn into the sample inlet by a capillary phenomenon,
the first flow channel is provided with an opening at a position opposite to the sample inlet which opening is closed in centrifugation, or a cross-sectional area of the air-discharging flow channel is set to be equal to or greater than the cross-sectional area of the first flow channel.

8. The centrifugal separation device according to claim 7, wherein an inner surface and the opening serving as the sample inlet of the first flow channel are rendered hydrophilic.

9. The centrifugal separation device according to claim 5, wherein
the divisional part is a joined body made up of a base member and a cover member,
the sample flow channel and the air-discharging flow channel are formed at a joining interface of the base member and the cover member, and
a space is formed adjacent to part of a surrounding of the third flow channel so that joining strength of the joining interface at the part of the surrounding of the third flow channel becomes weaker than joining strength of the joining interface at other part of the surrounding of the third flow channel.

10. The centrifugal separation device according to claims 1 to 9, wherein
the supporter is disc-like, a plurality of the divisional parts being radially disposed and connected onto a circumference of the disc, and
a portion connecting between the supporter and each of the divisional parts is formed to be thinner than, narrower than, or thinner and narrower than other part of each of the divisional parts.

11. The centrifugal separation device according to claims 1 to 9, wherein
the supporter is disc-like, on a surface of which disc recesses are formed for a plurality of the divisional parts to be respectively radially disposed and attached by being fitted into, and
the divisional parts and the recesses are shaped so as to prevent the divisional parts from shifting in a centrifugal force direction when the centrifugal force acts.

12. The centrifugal separation device according to claim 11, wherein the supporter is provided with a hole at a position corresponding to the extracting part when each of the divisional parts is attached.

13. The centrifugal separation device according to claims 1 to 12, wherein the supporter and the divisional parts are disc-like in a state where the divisional parts are mounted or attached to the supporter, and shaped in such a way that a plurality of the discs can be stored as being stacked.
